# EUROPEAN PATENT APPLICATION

(11) **EP 4 012 409 A1**
(43) Date of publication of application: **15.06.2022**
(21) Application number: 20212724.7
(22) Date of filing: 09.12.2020
(51) Int. Cl.: G01N 33/543

(54) **LINKER OF BIOPROBES**

(71) Applicant: Phoenix Silicon International Corp., Hsinchu 300 (TW)
(72) Inventor: LI, CHING-WEN, 300 Hsinchu (TW); CHEN, YU-CHE, 300 Hsinchu (TW)
(74) Representative: Lermer, Christoph

(57) **Abstract**

A linker of bioprobes, suitable for immobilizing a bioprobe on a chip substrate of a sensor, includes SH-(CH)n-NH2, SH-(CH)n-COOH, SH-(CH)n-SH, (OH)m-(CH)n-COOH or (OH)m-(CH)n-NH2, having a carbon number of 6 or more, m and n being integers greater than 1. When an average surface roughness (Ra) of the chip substrate is greater than 250 nm, coverage of the linker on the chip substrate is 40%-80%. A further linker of bioprobes, includes SH-(CH)n-NH2, SH-(CH)n-COOH, SH-(CH)n-SH, (OH)m-(CH)n-COOH or (OH)m-(CH)n-NH2, having a carbon number of less than 6, m and n being integers greater than 1. When an average surface roughness (Ra) of the chip substrate is less than 250 nm, coverage of the linker on the chip substrate is 65%-100%. The optimal carbon chain length of the linker and the coverage are realized for substrates of various roughnesses, and grasping ability of an electrochemical sensor chip for a detected object are enhanced.

## Description

### BACKGROUND

### Technical Field

The present invention relates to a linker compound, and more particularly to a linker of bioprobes.

### Related Art

In the manufacturing of an immune electrochemical biosensor, the number of bioprobes immobilized on a surface of a chip is related to the sensitivity of the chip, and the bioprobes immobilized on the surface of the chip are grafted through a linker for connecting the chip to the bioprobes. One end of the linker contains specific functional groups binding to different chemical reagents or small molecules. Therefore, how to promote the effective connection and coverage of the linker on the surface of the chip is a primary condition of manufacturing the biosensor. In the past, the connection and coverage of the linker were mostly focused on that the connection and coverage of the linker on the surface of a substrate reached a maximum amount. However, a practical condition was that even if the linker reached the maximum connection and coverage amount, a grasping amount of the chip on a detected object did not reach positive correlation.

To solve the above problems, the linker with a specific carbon chain length needs to be invented to be matched with a surface roughness of a biosensor chip and a coverage of the linker on the chip to obtain the maximum grasping amount for the detected object.

### SUMMARY

An objective of the present invention is to provide a linker of bioprobes, having a specific carbon chain length and capable of obtaining a maximum grasping amount for a detected object.

To achieve the above objective, the present invention provides a linker of bioprobes, suitable for immobilizing a bioprobe on a chip substrate of a sensor, including SH-(CH)n-NH2, SH-(CH)n-COOH, SH-(CH)n-SH, (OH)m-(CH)n-COOH or (OH)m-(CH)n-NH2, having a carbon number of 6 or more, m and n being integers greater than 1. When an average surface roughness (Ra) of the chip substrate is greater than 250 nm, a coverage of the linker on the chip substrate is in a range of 40% to 80%.

The present invention further provides a linker of bioprobes, suitable for immobilizing a bioprobe on a chip substrate of a sensor, including SH-(CH)n-NH2, SH-(CH)n-COOH, SH-(CH)n-SH, (OH)m-(CH)n-COOH or (OH)m-(CH)n-NH2, m and n are integers greater than 1. When an average surface roughness (Ra) of the chip substrate is less than 250 nm, a coverage of the linker on the chip substrate is in a range of 65% to 100%.

Compared with a conventional linker of biprobes, the present invention has the following advantages that the present invention has the optimal carbon chain length of the linker and the coverage for substrates of various roughnesses, and can greatly enhance the grasping ability of an electrochemical sensor chip for a detected object.

### BRIEF DESCRIPTION OF THE DRAWINGS

FIG. 1 to FIG. 2 are surface roughnesses of a gold (Au) substrate analyzed by atomic force microscopy (AFM).
FIG. 3 is a schematic diagram of performing a functional group activating reaction on a modified surface of a chip substrate.
FIG. 4 to FIG. 5 are surface roughnesses of the Au substrate analyzed by the AFM after modification.
FIG. 6 is a coverage of a linker of the present invention.
FIG. 7 is a schematic diagram of forming a peptide bond (-CO-NH-) between a bioprobe and a tail end of the linker to realize immobilization onto the surface of the Au substrate.
FIG. 8 to FIG. 9 are scanning electron microscope analysis photos of a grasping amount of a 3-mercaptopropionic acid (3-MPA) linker on odontoglossum ringspot viruses (ORSVs).
FIG. 10 is a scanning electron microscope analysis photo of a grasping amount of an 11-mercaptoundecanoic acid (11-MUA) linker on ORSVs.

### DETAILED DESCRIPTION

The present invention discloses a linker of bioprobes, capable of greatly enhancing the grasping ability of an electrochemical sensor chip for a detected object.

### Embodiment 1

A linker of bioprobes according to Embodiment 1 of the present invention, suitable for immobilizing a bioprobe on a chip substrate of a sensor, includes SH-(CH)n-NH2, SH-(CH)n-COOH, SH-(CH)n-SH, (OH)m-(CH)n-COOH or (OH)m-(CH)n-NH2, having a carbon number of 6 or more, m and n being integers greater than 1. When an average surface roughness (Ra) of the chip substrate is greater than 250 nm, a coverage of the linker on the chip substrate is in a range of 40% to 80%. The coverage is preferably in a range of 50%-70%.

A material of the chip substrate of the sensor of the present invention is silicon, glass, graphene, gold, platinum or polymers. The chip substrate using the gold or platinum material is suitable for roughness treatment by an acid cleaning manufacturing procedure. An acid cleaning solution may be used for surface treatment according to a proportion of 1 part of 98 wt% sulfuric acid and 3 parts of 33 wt% hydrogen peroxide, and a specific roughness is obtained by different treatment time. The chip substrate using other glass, ceramic or polymer materials may be subjected to roughening treatment by mechanical lapping, chemical agent etching or chemical mechanical lapping.

The roughened chip substrate is soaked into a solution of a linker dissolved in 99.8 wt% absolute alcohol and is placed at a room temperature to modify the surface of the chip substrate. Then, the modified surface of the chip substrate is subjected to functional group activating reaction by 1-ethyl-3-(3-dimethylaminopropyl)carbodiimide (EDC) or N-hydroxysuccinimide (NHS). Subsequently, the change of oxidation-reduction properties of the surface of the chip substrate may be measured by an electrochemical method. The coverage of modification molecules is further calculated through current density change.

The bioprobes of the present invention are bioprobes of enzymes, proteins, deoxyribonucleic acid (DNA), ribonucleic acid (RNA) or odontoglossum ringspot virus antibodies (ORSV antibodies).

### Embodiment 2

A linker of bioprobes according to Embodiment 2 of the present invention, suitable for immobilizing a bioprobe on a chip substrate of a sensor, includes SH-(CH)n-NH2, SH-(CH)n-COOH, SH-(CH)n-SH, (OH)m-(CH)n-COOH or (OH)m-(CH)n-NH2, having a carbon number of less than 6, m and n being integers greater than 1. When an average surface roughness (Ra) of the chip substrate is less than 250 nm, a coverage of the linker on the chip substrate is in a range of 65% to 100%. The coverage is preferably in a range of 80% to 100%.

Other technical contents of Embodiment 2 of the present invention are identical to those of Embodiment 1, including the materials of the chip substrate, the surface roughening treatment of the chip substrate, the linker solution modification, and the functional group activating reaction on the surface of the chip substrate. Additionally, types of suitable bioprobes are also identical.

Measured data of the linker of bioprobes of the present invention are as follows:

The present invention practically used pure gold as a substrate. A surface of the substrate was subjected to roughness change by acid cleaning. Then, C₃H₆O₂, 3-mercaptopropionic acid (3-MPA) and C₁₁H₂₂O₂S having different carbon chain lengths and having a thiol group (-SH) at one end and a carboxyl at one end were modified on the surface of the substrate. 11-mercaptoundecanoic acid (11-MUA) was used as a linker for immobilizing the bioprobe of ORSV antibody onto the surface, so as to facilitate grasping of a subsequent object of ORSVs as a practical example, and data were measured.

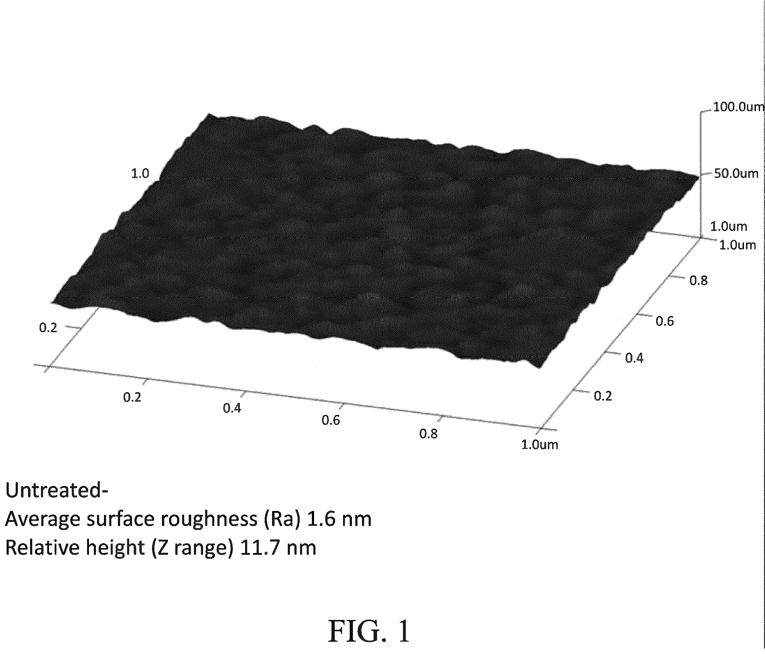

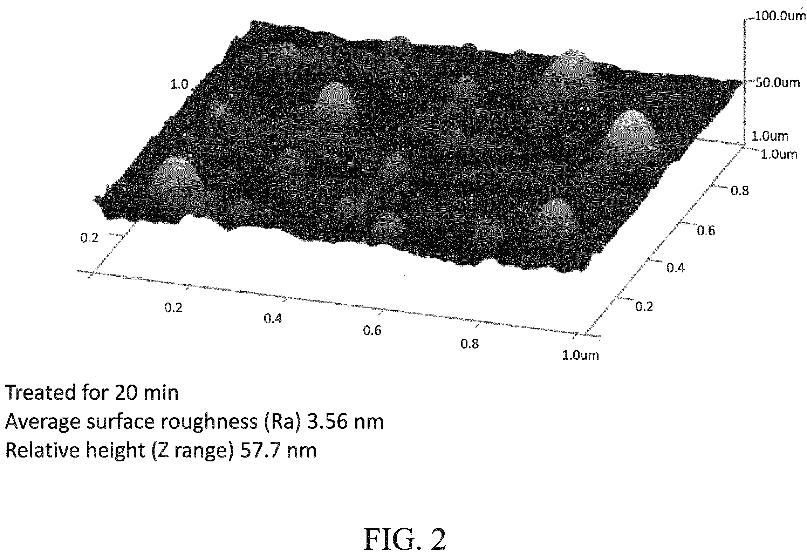

Treatment was performed on a surface of a gold (Au) substrate by 98 wt% sulfuric acid and 33 wt% hydrogen peroxide according to a proportion of 1:3, roughness change was generated through different treatment time, and the surface roughness of the Au substrate was analyzed by atomic force microscopy (AFM). As shown in FIG. 1, the average surface roughness (Ra) of an untreated (before treatment) surface was 1.6 nm, and a relative height (Z range) was 11.7 nm. As shown in FIG. 2, the average surface roughness (Ra) after acid cleaning for 20 min was 3.56 nm, and the relative height (Z range) was 57.7 nm.

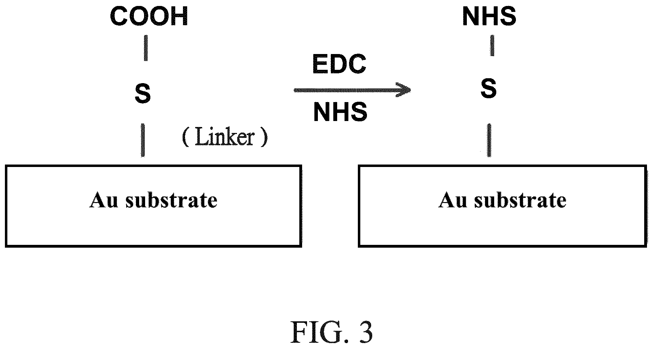

The roughened Au substrate was soaked into a 3-MPA and 11-MUA solution of 99.8 wt% absolute alcohol and was placed at a room temperature to modify the surface of the Au substrate. Then, the modified surface of the chip substrate was subjected to functional group activating reaction by 1-ethyl-3-(3-dimethylaminopropyl)carbodiimide (EDC) or N-hydroxysuccinimide (NHS), as shown in FIG. 3.

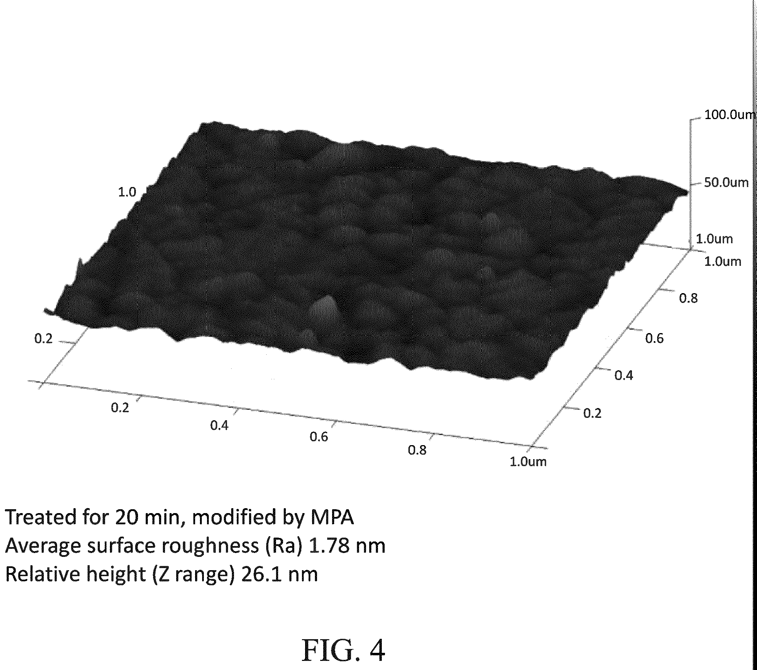

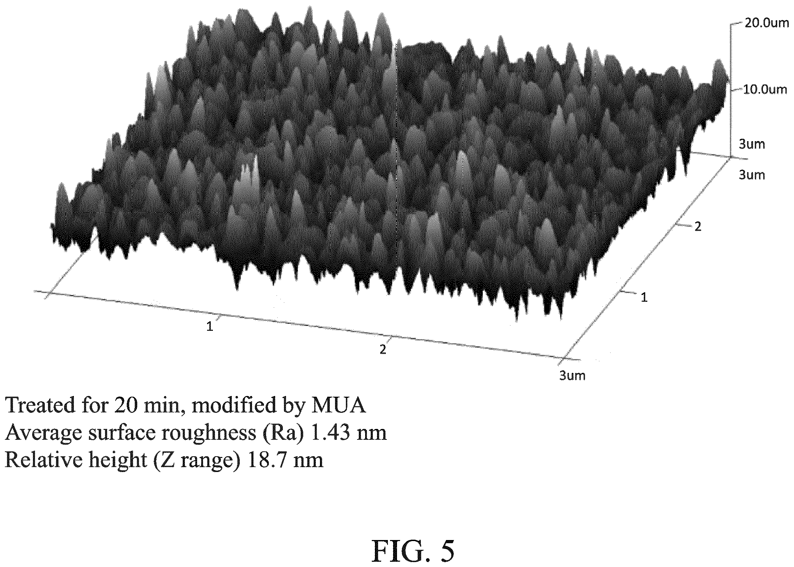

After acid cleaning treatment for 20 min, MPA modification was performed. Then, the surface roughness of the Au substrate was analyzed by the AFM. As shown in FIG. 4, the average surface roughness (Ra) was 1.78 nm, and the relative height (Z range) was 26.1 nm. After the acid cleaning treatment for 20 min, MUA modification was performed. Then, the surface roughness of the Au substrate was analyzed by the AFM. As shown in FIG. 5, the average surface roughness (Ra) was 1.43 nm, and the relative height (Z range) was 18.7 nm.

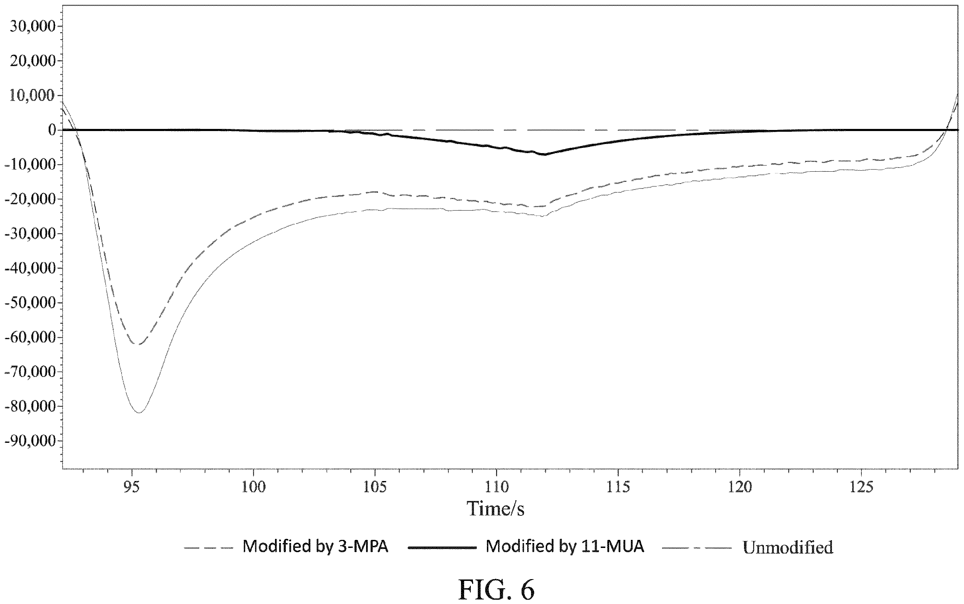

Subsequently, the change of oxidation-reduction properties of the surface of the chip substrate was measured by an electrochemical method. The coverage of the linker (modification molecules) was further calculated through current density change. As shown in FIG. 6, both 3-MPA and 11-MUA may fill surface roughnesses of the Au substrate. Through being reflected by an active area, it was known that the coverage of the 11-MUA was about 94.7%, and the coverage of the 3-MPA was about 20%.

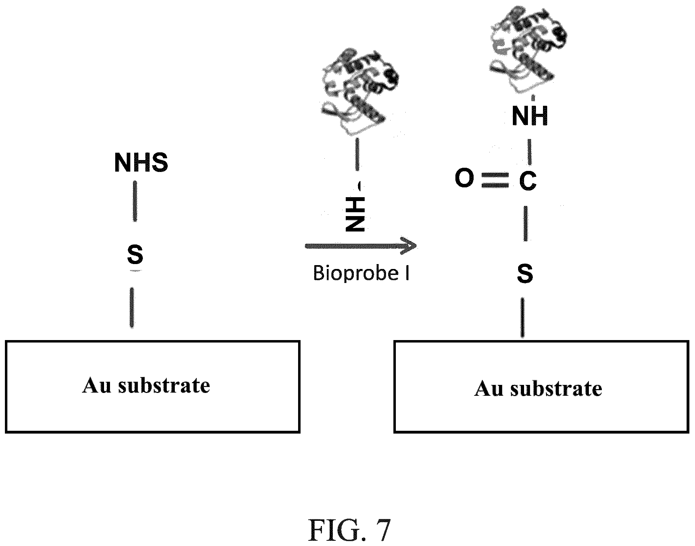

The bioprobe of ORSV antibody was dissolved in a buffer solution, and was dripped onto the MPA and MUA subjected to surface modification activation, so that a peptide bond (-CO-NH-) was formed between the bioprobe of the ORSV antibody and a tail end of the linker to realize immobilization onto the surface of the Au substrate. Then, the ORSV solution at the same concentration realized connection and coverage with the antibody, as shown in FIG. 7.

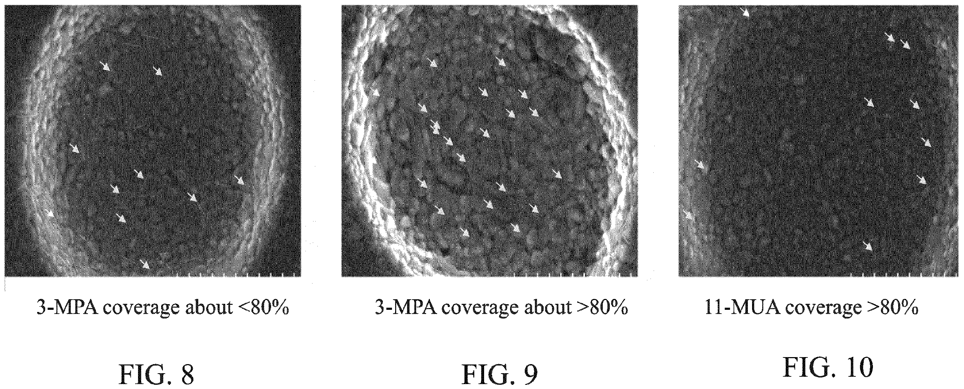

Finally, an ORSV grasping amount on the surface of the Au substrate was analyzed through scanning electron microscope (SEM) analysis. The results were as shown in FIG. 8 to FIG. 9. The ORSV grasping amount (FIG. 8) of a short-carbon-chain 3-MPA linker having the coverage of less than 80% and a carbon number of less than 6 was smaller than the ORSV grasping amount (FIG. 9) of a linker having the coverage of greater than 80%. That is, for the linker with a carbon number of less than 6, the ORSV grasping amount is greater if the coverage is greater. A result of the ORSV grasping amount of greater than 80% of the long-carbon-chain 11-MUA linker having the coverage of greater than 80% is as shown in FIG. 10. The ORSV grasping amount cannot be increased due to the greater coverage of a long carbon chain.

## Claims

1. A linker of bioprobes, suitable for immobilizing a bioprobe on a chip substrate of a sensor, comprising:
SH-(CH)n-NH2, SH-(CH)n-COOH, SH-(CH)n-SH, (OH)m-(CH)n-COOH or (OH)m-(CH)n-NH2, having a carbon number of 6 or more, m and n being integers greater than 1,
wherein when an average surface roughness (Ra) of the chip substrate is greater than 250 nm, a coverage of the linker on the chip substrate is in a range of 40% to 80%.

2. The linker of bioprobes according to claim 1, wherein a material of the chip substrate of the sensor is silicon, glass, graphene, gold, platinum or polymers.

3. The linker of bioprobes according to claim 1, wherein the chip substrate is subjected to surface treatment according to a proportion of 1 part of 98 wt% sulfuric acid and 3 parts of 33 wt% hydrogen peroxide, and a specific roughness is obtained by different treatment time.

4. The linker of bioprobes according to claim 1, further comprising: soaking the chip substrate into a solution of a linker dissolved in 99.8 wt% absolute alcohol, placing at a room temperature to modify the surface of the chip substrate, and then, subjecting the surface of the chip substrate to functional group activating reaction by 1-ethyl-3-(3-dimethylaminopropyl) carbodiimide (EDC) or N-hydroxysuccinimide (NHS).

5. The linker of bioprobes according to claim 1, wherein the bioprobes are bioprobes of enzymes, proteins, deoxyribonucleic acid (DNA), ribonucleic acid (RNA) or odontoglossum ringspot virus antibodies (ORSV antibodies).

6. A linker of bioprobes, suitable for immobilizing a bioprobe on a chip substrate of a sensor, comprising:
SH-(CH)n-NH2, SH-(CH)n-COOH, SH-(CH)n-SH, (OH)m-(CH)n-COOH or (OH)m-(CH)n-NH2, having a carbon number of less than 6, m and n being integers greater than 1,
wherein when an average surface roughness (Ra) of the chip substrate is less than 250 nm, a coverage of the linker on the chip substrate is in a range of 65% to 100%.

7. The linker of bioprobes according to claim 6, wherein a material of the chip substrate of the sensor is silicon, glass, graphene, gold, platinum or polymers.

8. The linker of bioprobes according to claim 6, wherein the chip substrate is subjected to surface treatment according to a proportion of 1 part of 98 wt% sulfuric acid and 3 parts of 33 wt% hydrogen peroxide, and a specific roughness is obtained by different treatment time.

9. The linker of bioprobes according to claim 6, further comprising: soaking the chip substrate into a solution of a linker dissolved in 99.8 wt% absolute alcohol, placing at a room temperature to modify the surface of the chip substrate, and then, subjecting the surface of the chip substrate to functional group activating reaction by 1-ethyl-3-(3-dimethylaminopropyl) carbodiimide (EDC) or N-hydroxysuccinimide (NHS).

10. The linker of bioprobes according to claim 6, wherein the bioprobes are bioprobes of enzymes, proteins, deoxyribonucleic acid (DNA), ribonucleic acid (RNA) or odontoglossum ringspot virus antibodies (ORSV antibodies).
